# EUROPEAN PATENT APPLICATION

(11) **EP 2 671 589 A1**
(43) Date of publication of application: **11.12.2013**
(21) Application number: 12742277.2
(22) Date of filing: 01.02.2012
(51) Int. Cl.: A61K 31/499, A61K 45/00, A61P 9/00, A61P 27/02

(54) **MEDICINAL AGENT FOR PREVENTION OR TREATMENT OF DISEASES ASSOCIATED WITH INTRAOCULAR NEOVASCULARIZATION AND/OR INTRAOCULAR VASCULAR HYPERPERMEABILITY**

(30) Priority: 02.02.2011 JP 2011020270
(71) Applicant: Public University Corporation Nagoya City University, Mizuho-ku Nagoya-shi Aichi 467-8601 (JP); Sanwa Kagaku Kenkyusho Co., Ltd, Nagoya-shi Aichi 461-8631 (JP)
(72) Inventor: OGURA Yuichiro, Nagoya-shi Aichi 467-8601 (JP); NOZAKI Miho, Nagoya-shi Aichi 467-8601 (JP); NAKAJIMA Atsushi, Nagoya-shi Aichi 461-8631 (JP); HIBI Chihiro, Nagoya-shi Aichi 461-8631 (JP)
(74) Representative: Duckworth, Timothy John
(86) International application number: PCT/JP2012/052288
(87) International publication number: WO 2012/105610

(57) **Abstract**

A pharmaceutical for preventing or treating a disorder accompanied by ocular angiogenesis and/or increased ocular vascular permeability, composed of a combination of an anti-VEGF agent, and a pyrrolo[1,2-a]pyrazine derivative represented by a general formula (I): 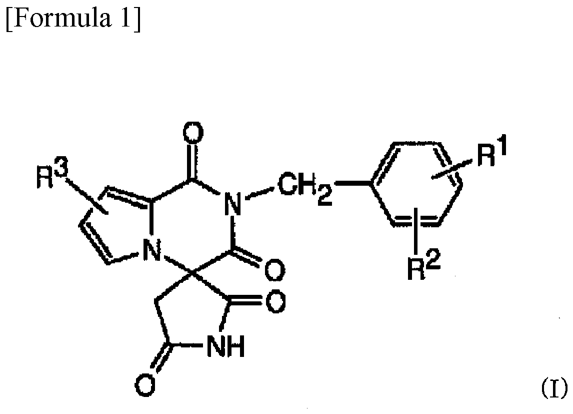 in the formula, R¹ and R² are simultaneously or individually each a hydrogen atom, a halogen atom, a trifluoromethyl group, a lower alkyl group, a lower alkoxy group or a nitro group, and R³ is a hydrogen atom, a halogen atom or a lower alkyl group, or a pharmacologically acceptable salt thereof.

## Description

### Technical Field

The present invention relates to a pharmaceutical for preventing or treating a disorder accompanied by ocular angiogenesis and/or increased ocular vascular permeability, which is composed of a combination of an anti-VEGF agent and a specific pyrrolo[1,2-a]pyrazine derivative.

### Background Art

Vascular endothelial growth factor (hereinafter referred to as VEGF) is the most responsible for angiogenesis and/or increased ocular vascular permeability. It was clarified in recent years that VEGF is responsible for various ophthalmic lesions based on physiological actions such as angiogenesis, increased vascular permeability and proliferation of vascular endothelial cells.

Pharmaceuticals targeting VEGF have been already used widely in clinical use for the treatment of age-related macular degeneration (AMD), branch retinal vein occlusion (BRVO), central retinal vein occlusion (CRVO), diabetic maculopathy, diabetic retinopathy, neovascular glaucoma or the like. Examples of anti-VEGF agents may include receptor antagonists, anti-VEGF antibodies, VEGF Ligand inhibitors and nucleic acid drugs, which relate to VEGF, and the like. Specific examples include bevacizumab sodium, sorafenib, sunitinib, pegaptanib sodium, ranibizumab, aflibercept (VEGF-Trap EYE) and the like. These are generally used as intravitreal injections in the field of ophthalmology.

(3R)-2' -(4-bromo-2-fluorobenzyl)spiro[pyrrolidine-3,4' (1'H)pyr rolo[1,2-a]pyrazine]-1',2, 3', 5(2'H)-tetrone (ranirestat) is an aldose reductase (AR) inhibitor. For this compound, use as a therapeutic agent for diabetic complications such as cataract, neuropathy and nephropathy is known (Patent Documents 1 and 2), but the angiogenesis suppressing effect and the like are not known. For specific kinds among aldose reductase (AR) inhibitors, uses relating to age-related macular degeneration, branch retinal vein occlusion, central retinal vein occlusion and the like (Patent Documents 3 and 4) are suggested.

It is known that anti-VEGF agents are used in combination with other pharmaceuticals (Patent Documents 5 and 6). Combination use with Tumor Necrosis Factor α (TNFα) (Patent Documents 7 and 8), combination use with a receptor-type tyrosine kinase inhibitor (Patent Document 9) and combination use with an antihypertensive (Patent Document 10) are reported. Furthermore, combination use of an anti-VEGF aptamer, which is an anti-VEGF agent, with other anti-VEGF agent (Patent Document 11) is reported. However, combination use of an anti-VEGF agent with a pyrrolo[1,2-a]pyrazine derivative has not been reported yet.

### Citation List

### Patent Literature

Patent Document 1: Japanese Patent No. 2516147
Patent Document 2: Japanese Patent No. 3158128
Patent Document 3: Japanese Patent Application Laid-Open Publication No. 11-310538
Patent Document 4: International Publication No. W02008/093691
Patent Document 5: Japanese Patent Application National Publication (Laid-Open) No. 2007-505938
Patent Document 6: Japanese Patent Application National Publication (Laid-Open) No. 2007-505939
Patent Document 7: Japanese Patent Application National Publication (Laid-Open) No. 2004-529149
Patent Document 8: Japanese Patent Application Laid-Open Publication No. 2009-256373
Patent Document 9: Japanese Patent Application Laid-Open Publication No. 2009-102359
Patent Document 10: Japanese Patent Application National Publication (Laid-Open) No. 2008-537538
Patent Document 11: Japanese Patent Application National Publication (Laid-Open) No. 2005-511576

### Summary of Invention

### Technical Problem

Anti-VEGF agents that are widely used in clinical use for disorders accompanied by ocular angiogenesis and/or increased ocular vascular permeability are in dosage forms for injecting into the vitreum of a subject patient, and frequent intravitreal injection is required.

However, intravitreal injection provides a heavy emotional, physical and medical-economic burden on patients and medical clinics, and thus tends to lead to the interruption of the treatment. Furthermore, when the dose is increased in a patient having low satisfaction with treatment, side effects such as stroke tend to be easily caused. For example, it is reported that ranibizumab, which is a typical anti-VEGF agent, has a short half-life and has an elimination half-life of 9 days when it is injected into the vitreous body of a human (the package insert of ranibizumab). This leads to the increase in the frequency of administration of ranibizumab, and is a great burden on patients and medical clinics.

Accordingly, a strategy for enhancing the clinical effect of an anti-VEGF agent and a strategy for decreasing the frequency or dose are required. Therefore, the present invention aims at decreasing the dosage and frequency of administration of an anti-VEGF agent and improving the effectiveness thereof.

### Solution to Problem

In view of the above-mentioned problem, the present inventors have done intensive studies and found that these objects can be achieved by combining an anti-VEGF agent and a pyrrolo[1,2-a]pyrazine derivative represented by the following general formula (I), and completed the invention. Namely, the major constitutions of the present invention are as follows.

(1) A pharmaceutical for preventing or treating a disorder accompanied by ocular angiogenesis and/or increased ocular vascular permeability composed of a combination of an anti-VEGF agent and a pyrrolo[1,2-a]pyrazine derivative represented by a general formula (I): 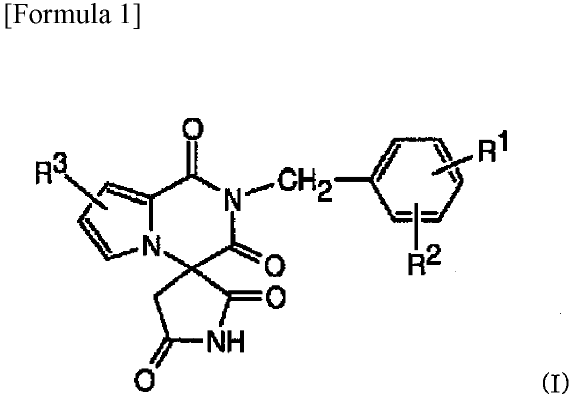 in the formula, R¹ and R² are simultaneously or individually each a hydrogen atom, a halogen atom, a trifluoromethyl group, a lower alkyl group, a lower alkoxy group or a nitro group, and R³ is a hydrogen atom, a halogen atom or a lower alkyl group, or a pharmacologically acceptable salt thereof.

(2) A pharmaceutical for preventing or treating a disorder accompanied by ocular angiogenesis and/or increased ocular vascular permeability, comprising a pyrrolo[1,2-a]pyrazine derivative represented by a general formula (I): 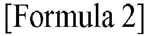 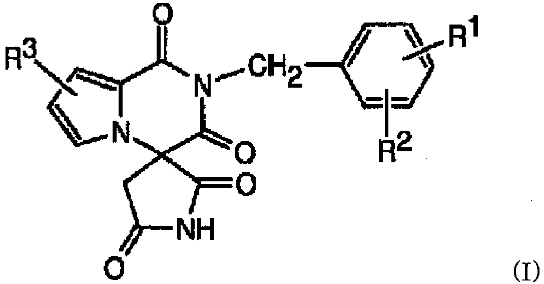 in the formula, R¹ and R² are simultaneously or individually each a hydrogen atom, a halogen atom, a trifluoromethyl group, a lower alkyl group, a lower alkoxy group or a nitro group, and R³ is a hydrogen atom, a halogen atom or a lower alkyl group, or a pharmacologically acceptable salt thereof as an active ingredient, and the pharmaceutical being administered in combination with an anti-VEGF agent.

(3) The pharmaceutical according to (1) or (2), wherein the pyrrolo[1,2-a]pyrazine derivative represented by the general formula (I) is
(3R)-2'-(4-bromo-2-fluorobenzyl)spiro[pyrrolidine-3,4'(1'H)pyrrolo[1, 2-a]pyrazine]-1',2,3',5(2'H)-tetrone.

(4) The pharmaceutical according to any one of (1) to (3), wherein the anti-VEGF agent is an intravitreal injection drug.

(5) The pharmaceutical according to (4), wherein the intravitreal injection is selected from the group consisting of an anti-VEGF antibody, a VEGF Ligand inhibitor, a VEGF receptor antagonist and a nucleic acid drug related to VEGF.

(6) The pharmaceutical according to any one of (1) to (5), wherein the disorder accompanied by ocular angiogenesis and/or increased ocular vascular permeability is selected from the group consisting of age-related macular degeneration (AMD), branch retinal vein occlusion (BRVO), central retinal vein occlusion (CRVO), diabetic maculopathy, diabetic retinopathy and neovascular glaucoma.

(7) A kit comprising:
the pharmaceutical according to (2); and
a written instruction describing a method for administering the pharmaceutical in combination with an anti-VEGF agent for preventing or treating a disorder accompanied by ocular angiogenesis and/or increased ocular vascular permeability.

### Advantageous Effects of Invention

The pharmaceutical of the present invention can enhance an action of suppressing ocular angiogenesis and/or an action of suppressing increased ocular vascular permeability and can maintain those effects, in ocular disorders to which an anti-VEGF agent is administered. Alternatively, the pharmaceutical of the present invention enables decrease the dose or enhance the medicinal effect of the anti-VEGF agent, and extension of the intervals of intravitreal re-injection, decreasing of the frequency of intravitreal injection, extension of the administration intervals, by maintaining the prolonged medicinal effect. Furthermore, since the pyrrolo[1,2-a]pyrazine derivative represented by the general formula (I) can be orally administered, it has an advantage that the derivative is easily used, and thus it is an administration method that provides little burden on patients and medical clinics.

### Brief Description of Drawings

FIG. 1 is a graph showing the choroidal neovascularization volume (CNV) at 7 days after the irradiation of laser. The "*" in the drawing represents the presence of a significant difference at a risk rate of 5%.
FIG. 2 is a graph showing the CNV volume at 7 days after the irradiation of laser. The "***" in the drawing represents the presence of a significant difference at a risk rate of 0.1%.

### Description of Embodiments

Hereinafter the embodiments of the present invention will be explained in more detail. However, the present invention is not construed to be limited by the following embodiments.

The pyrrolo[1,2-a]pyrazine derivative represented by the general formula (I) or a pharmacologically acceptable salt thereof in the present invention is a compound having an aldose reductase inhibitory activity, and can be produced according to the synthesis method described in Japanese Patent No. 2516147 or the like.

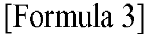

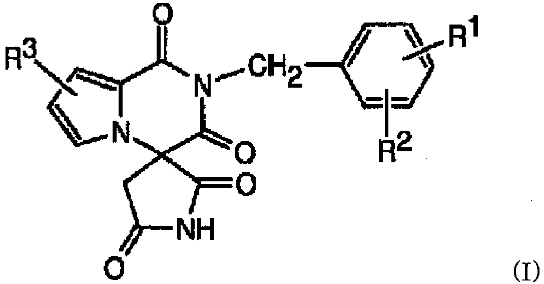

In the formula, R¹ and R² are simultaneously or individually each a hydrogen atom, a halogen atom, a trifluoromethyl group, a lower alkyl group, a lower alkoxy group or a nitro group, and R³ means a hydrogen atom, a halogen atom or a lower alkyl group. As the lower alkyl group, C₁ to C₃ alkyl groups are preferable, of which a methyl group is preferable. As the lower alkoxy group, C₁ to C₃ alkoxy groups are preferable, of which a methoxy group is preferable. Among the compounds of the general formula (I), (3R)-2'-(4-bromo-2-fluorobenzyl)spiro[pyrrolidine-3,4'(1'H)pyrrolo[1, 2-a]pyrazine]-1', 2, 3', 5(2'H)-tetrone is preferable.

The pyrrolo[1,2-a]pyrazine derivative represented by the general formula (I) or a pharmacologically acceptable salt thereof is administered in combination with an anti-VEGF agent for preventing or treating a disorder accompanied by ocular angiogenesis and/or increased ocular vascular permeability. In other words, the pharmaceutical comprising a pyrrolo[1,2-a]pyrazine derivative represented by the general formula (I) or a pharmacologically acceptable salt thereof as an active ingredient is used for an agent for enhancing the effect of the anti-VEGF agent for the prevention or treatment of a disorder accompanied by ocular angiogenesis and/or increased ocular vascular permeability.

In still other words, the pharmaceutical according to the present invention is a pharmaceutical that is constituted by a combination of an anti-VEGF agent and a pyrrolo[1,2-a]pyrazine derivative represented by the general formula (I) or a pharmacologically acceptable salt thereof. This pharmaceutical is also administered for preventing or treating a disorder accompanied by ocular angiogenesis and/or increased ocular vascular permeability.

VEGF is a group of sugar proteins that are responsible for vasculogenesis and angiogenesis, and is produced due to various causes such as aging, ischemic reperfusion and inflammation. When VEGF binds to a vascular endothelial cell growth factor receptor (a VEGF receptor), vascular endothelial cells are activated. The activated vascular endothelial cells migrate and proliferate to neovascularization. Furthermore, VEGF increases vascular permeability and causes hemorrhage, leakage of the components in blood and pooling of exudates.

The anti-VEGF agent inhibits a vascular endothelial growth factor (VEGF) signaling pathways, or inhibits the biological activity of VEGF. The anti-VEGF agent shows pharmacological actions such as suppression of increased angiogenesis, suppression of increased vascular permeability and suppression of the growth of vascular endothelial cells by attenuating the activity of VEGF.

Examples of the anti-VEGF agents may include anti-VEGF antibodies, VEGF Ligand inhibitors, VEGF receptor antagonists and nucleic acid drugs related to VEGF. The VEGF receptor antagonists antagonize VEGF to inhibit the binding of the VEGF to VEGF receptors. Anti-VEGF antibodies, VEGF Ligand inhibitors and some of nucleic acid drugs related to VEGF inhibit the binding of VEGF to VEGF receptors by forming a conjugate with VEGF. Other nucleic acid drugs related to VEGF inhibit the synthesis of VEGF-related proteins. The anti-VEGF antibodies include anti-VEGF antibodies obtained by humanizing mouse anti-VEGF antibodies by gene recombination, fragments thereof, or those obtained by modifying a part of the amino acid sequences thereof. The VEGF Ligand inhibitors include human-type VEGF receptor fusion proteins.

The anti-VEGF antibodies, VEGF Ligand inhibitors, VEGF receptor antagonists and nucleic acid drugs related to VEGF are common in that all of them have an action of suppressing VEGF signal transmission. As the other anti-VEGF agent, an agent that directly suppresses the production and/or synthesis of VEGF can be considered.

Specific examples of the VEGF Ligand inhibitors include aflibercept (VEGF-Trap EYE). Specific examples of the anti-VEGF antibodies include bevacizumab sodium, ranibizumab, sorafenib and sunitinib. Specific examples of the nucleic acid drugs related to VEGF include pegaptanib sodium that is an aptamer, and RTP801i-14 that is siRNA. These are used as intravitreal injections. In addition, bevacizumab sodium, ranibizumab and VEGF-Trap EYE and the like are characterized by that they inhibit the binding of VEGF isoforms to receptors by widely binding to all VEGF isoforms in a non-selective manner. On the other hand, pegaptanib sodium is characterized by that it selectively binds to VEGF165 that is the most deeply involved in pathologic intraocular neovascularization.

The medicinal effect that is exerted by administering the pyrrolo[1,2-a]pyrazine derivative of the general formula (I) or a salt thereof and the anti-VEGF agent in combination is within substantially the same of the medicinal effect that is exerted by administering the anti-VEGF agent alone. The major pharmacological actions of the anti-VEGF agent are an action of suppressing angiogenesis and an action of suppressing increased vascular permeability (an action of suppressing edema). Therefore, the scope of the application of the anti-VEGF agent refers to ocular disorders accompanied by ocular angiogenesis and/or increased ocular vascular permeability, and an exemplary embodiment of the pharmaceutical according to the present invention is effective for those ocular disorders.

Examples of the ocular disorders accompanied by ocular neovascularization may include ocular angiogenic disorders such as wet age-related macular degeneration (wet-AMD), branch retinal vein occlusion (BRVO), central retinal vein occlusion (CRVO), diabetic retinopathy, neovascular glaucoma, myopic choroidal neovascularization, retinitis pigmentosa, retinopathy of prematurity and secondary choroidal neovascularization.

Another exemplary embodiment of the pharmaceutical according to the present invention is a pharmaceutical that is also effective for disorders accompanied by increased ocular vascular permeability. Examples of the disorders accompanied by increased ocular vascular permeability may include wet age-related macular degeneration (wet-AMD), branch retinal vein occlusion (BRVO), central retinal vein occlusion (CRVO), diabetic maculopathy, retinitis pigmentosa, diabetic retinopathy and edema due to retinal photocoagulation (panretinal photocoagulation, grid photocoagulation, Grid Pattern photocoagulation). The ocular angiogenic disorders and disorders accompanied by increased ocular vascular permeability are not limited to these disorders. The pharmaceutical according to the present invention shows a synergistic medicinal effect on these disorders accompanied by ocular angiogenesis and/or increased ocular vascular permeability for which the administration of the anti-VEGF agent is considered to be effective.

Age-related macular degeneration is a disorder caused by the degeneration in the macula associated with aging, and is classified into "wet" and "dry" according to the presence or absence of neovascularization that are generated from the choroid (choroidal neovascularization). The pharmaceutical according to the present invention is specifically effective for wet age-related macular degeneration.

Wet age-related macular degeneration is caused by generation of abnormal choroidal neovascularization (CNV) on sites centered around a macular area. These neovascular vessels may cause hemorrhage or exudation (increased vascular permeability), degenerate the tissue in the macular area relating to visual acuity, cause a malfunction and lead to scarring of the tissue. It is considered that VEGF is strongly involved in intraocular neovascular vessels.

Diabetic maculopathy is a generic term that includes impairment in the macular area caused by macular edema due to increased vascular permeability, and impairments in the macular area caused by occlusion of capillary vessels or impairment of pigment epithelial cells. The major lesion is macular edema. Since the amounts of VEGF are high in the eyes of a patient with macular edema and VEGF has an extremely strong action of elevating vascular permeability, it is considered that VEGF are strongly involved in macular edema in diabetic maculopathy.

Retinal vein occlusion (RVO) includes branch retinal vein occlusion (BRVO) and central retinal vein occlusion (CRVO). VEGF is produced due to vein occlusion, and edema and angiogenesis are formed mainly on macular areas. RVO is one of disorders accompanied by ocular angiogenesis and/or increased ocular vascular permeability, and the involvement of VEGF has been reported.

Furthermore, it is considered that ocular angiogenesis and increased ocular vascular permeability are also responsible for the onset and/or progress of retinal disorders such as central exudative chorioretinopathy, angioid streaks, retinal pigment epithelial detachment, multifocal choroiditis, choroidal neovascularization and retinopathy of prematurity.

The pyrrolo[1,2-a]pyrazine derivative of the general formula (I) or a salt thereof and the anti-VEGF agent, which are used as active ingredients of the pharmaceutical according to the present invention, may be incorporated respectively in plural separate formulations, or may be incorporated in the same formulation as one formulation. The "pharmaceutical composed of a combination (pharmaceutical comprising a combination)" is a pharmaceutical in which plural drugs or the active ingredients thereof are administered in combination, in other words, administered by combination use.

In the case when plural active ingredients to be administered are incorporated in plural separate formulations, these formulations are not necessarily administered at the same time to a patient. According to such administration by combination use, the effect of the combination use can be brought out sufficiently even when one of the pharmaceuticals (or the active ingredient thereof) does not maintain an effective concentration in blood or an effective concentration in tissue; therefore, it is not always necessary to use an administration method in which the terms in which the respective pharmaceuticals maintain their effective concentrations in blood or effective concentrations in tissue are overlapped. In general, since the respective formulations are administered according to their original respective administration methods, the formulations may be administered by the same frequency or different frequencies.

Furthermore, the active ingredients thereof can respectively have different dosage forms. Namely, the dosage form of the anti-VEGF agent is an intravitreal injection, and the dosage form of the pyrrolo[1,2-a]pyrazine derivative represented by the general formula (I) is an oral agent, but the dosage forms are not limited to these forms. The pharmaceutical comprising the pyrrolo[1,2-a]pyrazine derivative represented by the general formula (I) or a pharmacologically acceptable salt thereof as an active ingredient is used as an agent for enhancing the effect of the anti-VEGF agent to prevent or treat disorders accompanied by ocular angiogenesis and/or increased ocular vascular permeability, or as an agent for sustaining the effect of the anti-VEGF agent.

Examples of the administration manners of the above-mentioned pharmaceuticals may be those as listed below:
(1) administration of a single formulation comprising both the anti-VEGF agent and the pyrrolo[1,2-a]pyrazine derivative represented by the general formula (I),
(2) simultaneous administration of two kinds of formulations obtained by separately formulating the anti-VEGF agent and the pyrrolo[1,2-a]pyrazine derivative represented by the general formula (I) in the same administration route,
(3) staggered administration of two kinds of formulations obtained by separately formulating the anti-VEGF agent and the pyrrolo[1,2-a]pyrazine derivative represented by the general formula (I) in the same administration route,
(4) simultaneous administration of two kinds of formulations obtained by separately formulating the anti-VEGF agent and the pyrrolo[1,2-a]pyrazine derivative represented by the general formula (I) in different administration routes,
(5) staggered administration of two kinds of formulations obtained by separately formulating the anti-VEGF agent and the pyrrolo[1,2-a]pyrazine derivative represented by the general formula (I) in different administration routes,
(6) administration of two kinds of formulations obtained by separately formulating the anti-VEGF agent and the pyrrolo[1,2-a]pyrazine derivative represented by the general formula (I) in the same administration route at different administration intervals, and
(7) administration of two kinds of formulations obtained by separately formulating the anti-VEGF agent and the pyrrolo[1,2-a]pyrazine derivative represented by the general formula (I) in different administration routes at different administration intervals.

In addition, as the administration manner of (5), administration in the order that the anti-VEGF agent is first administered and then the pyrrolo[1,2-a]pyrazine derivative represented by the general formula (I) such as ranirestat is administered after a certain time period has passed, or administration in the inverse order can be exemplified. As the administration manner of (6) or (7), administration by the following administration intervals may be exemplified: administration by first administrating the anti-VEGF agent and then administrating the pyrrolo[1,2-a]pyrazine derivative represented by the general formula (I) every day until the next administration of the anti-VEGF agent, or administration by first administrating the pyrrolo[1,2-a]pyrazine derivative represented by the general formula (I) every day, then administrating the anti-VEGF agent, and subsequently administrating the pyrrolo[1,2-a]pyrazine derivative described above every day.

The pharmaceutical according to the present invention is, whether it is a pharmaceutical comprising a plurality of active ingredients in a plurality of separate formulations or a single formulation including the active ingredients in the same formulation, formulated into, for example, a tablet, a capsule agent, a powder, a granular agent, a liquid agent or a syrup agent by a general formulation technique, in an acceptable dosage form.

In the case of a solid agent, excipients that are pharmacologically acceptable in formulation, for example, starch, lactose, purified white soft sugar, glucose, crystalline cellulose, carboxycellulose, carboxymethyl cellulose, carboxyethyl cellulose, calcium phosphate, magnesium stearate and gum arabic can be used, and where necessary, a lubricant, a binder, a disintegrating agent, a coating agent, a colorant and the like can be incorporated. In the case of a liquid agent, a stabilizer, a dissolution aid, a suspending agent, an emulsifying agent, a buffer agent, a preservative and the like can be used.

The dose of the pharmaceutical according to the present invention can be suitably selected according to the general dose of the anti-VEGF agent or the pyrrolo[1,2-a]pyrazine derivative represented by the general formula (I) depending on the subject to be administered, administration route, intended disorder, dosage form and the like. Namely, the dose of the pyrrolo[1,2-a]pyrazine derivative represented by the general formula (I), specifically ranirestat, differs depending on the symptom, age, method for administration, dosage form and the like, and generally 10 to 160 mg/day, preferably 40 to 80 mg/day is administered once or in portions each day per one adult human patient (body weight: 60 kg). The dose in the case of oral administration is generally 10 to 160 mg/day, preferably 20 to 80 mg/day, further preferably 40 to 80 mg/day per one adult human patient (body weight: 60 kg).

The dose of the anti-VEGF agent differs depending on the agent. The dose of bevacizumab sodium is from 0.1 mg/time to 2.5 mg/time per an adult patient by intravitreal administration. The dose of pegaptanib is generally from 0.1 mg/time to 3 mg/time, preferably 0.3 mg/time per an adult patient by intravitreal administration. The dose of ranibizumab is generally from 0.1 mg/time to 0.5 mg/time, preferably 0.5 mg/time per an adult patient by intravitreal administration. The dose of VEGF-TRAP EYE is generally from 0.1 mg/time to 4 mg/time per an adult patient by intravitreal administration. The anti-VEGF agent is essentially administered once a month to once two to three months in the case of intravitreal administration. Alternatively, the agent is administered as necessary in the case when the lesion deteriorates or relapses. Although the usage and dosage are general usage and dosage of the anti-VEGF agent in the case when the anti-VEGF agent is not administered in combination with the pyrrolo[1,2-a]pyrazine derivative of the general formula (I) and the like, in the case when the anti-VEGF agent is administered in combination with the pyrrolo[1,2-a]pyrazine derivative of the general formula (I) and the like, the dose of the anti-VEGF agent can be decreased and the frequency of administration can be decreased with consideration for enhancing of the effect by the combination.

The pharmaceutical of the present invention can also constitute a kit by the pharmaceutical comprising the pyrrolo[1,2-a]pyrazine derivative of the general formula (I) and the like, and a written instruction that describes a method for administering the pharmaceutical in combination with the anti-VEGF agent for preventing or treating a disorder accompanied by ocular angiogenesis and/or increased ocular vascular permeability. The written instruction in the kit includes descriptive texts regarding the usage and dosage and the like in the case when the pharmaceutical comprising the pyrrolo[1,2-a]pyrazine derivative of the general formula (I) is administered in combination with the anti-VEGF agent. The usage and dosage thereof are as mentioned above.

### Examples

The present invention will be explained in more detail by the following experimental examples. However, these do not limit the present invention. The present invention may be carried out by an embodiment that is modified to the extent that it does not deviate from the intent of the present invention.

Using a mouse model in which choroidal neovascularization (CNV) are experimentally induced by laser irradiation, the present inventors evaluated the effectiveness against the volume of CNV. In the CNV model, macrophages migrate by the initiation of an inflammation by laser irradiation, and as a result thereof, VEGF is produced, neovascular vessels are generated, and increased vascular permeability is observed. In other words, the CNV model is an experimental model that shows effectiveness against disorders accompanied by ocular angiogenesis and/or increased ocular vascular permeability through VEGF.

### Experiment 1:

### Method

The both eyes of a male C57BL/6J mice having a body weight of about 20 g (6-week old) were irradiated with laser (irradiated by 4 to 6 spots per one eye) to experimentally induce CNV. As mentioned below, the drugs were administered by an anti-VEGF antibody alone and ranirestat alone, respectively, or by a combination of the two drugs. The dose of ranirestat was 3 mg/kg/day, which is a general dosage used in literature. As the anti-VEGF antibody, a mouse anti-VEGF antibody purchased from R&D Systems was used. The number of examples was five per a group, and the following four groups were set.
(a) Untreated group
(b) 3 mg/kg ranirestat-administered group: 3 mg/kg of ranirestat was forcibly and orally administered once a day (3 mg/kg/day)
(c) Anti-VEGF antibody-administered group: 1ng of an anti-VEGF antibody was administered by intravitreal injection
(d) Combination use group: 1ng of an anti-VEGF antibody was administered by intravitreal injection + ranirestat was administered (3 mg/kg was forcibly and orally administered once a day (3 mg/kg/day))

Ranirestat was administered from the days of the laser irradiation (20 to 30 minutes after the irradiation) to 7 days after the irradiation. The anti-VEGF antibody was administered by intravitreal injection immediately after the laser irradiation. After completion of the laser irradiation, the mice were bred by general breeding for 7 days, and thereafter the eye balls were excised and the volume of CNV appeared in the choroid was measured. The induction and evaluation of CNV were conducted by the following procedures.

### [Induction of experimental CNV by laser irradiation]

Each mouse was anesthetized by administering 0.30 mL of tribromoethanol to the abdominal cavity of the mouse, and a mydriatics and a local anesthesia were placed a drop of the eyes. Thereafter the both eyes of the mouse were irradiated with laser (wavelength: 532 nm, output: 200 mW, irradiation time: 100 ms, spot size: 100 µm) by 4 to 6 spots per one eye using a laser photocoagulation device (Lumenis). CNV appeared by the laser irradiation, and thereafter the CNV volume increased.

### [Evaluation of CNV volume]

At 7 days after the laser irradiation, each mouse was euthanized under tribromoethanol anesthesia, and the left and right eye balls of the mouse were excised. Using the excised eye balls, flat mounts were prepared according to a conventional method. CNV was stained by using FITC-Griffonia simplicifolia isolectin-B4. The volume of CNV was measured by importing an image in the vicinity of CNV by a confocal laser microscope (Zeiss LSM5 Pascal) and using image analysis software (NIH Image J). The volume was represented by µm³.

### Results

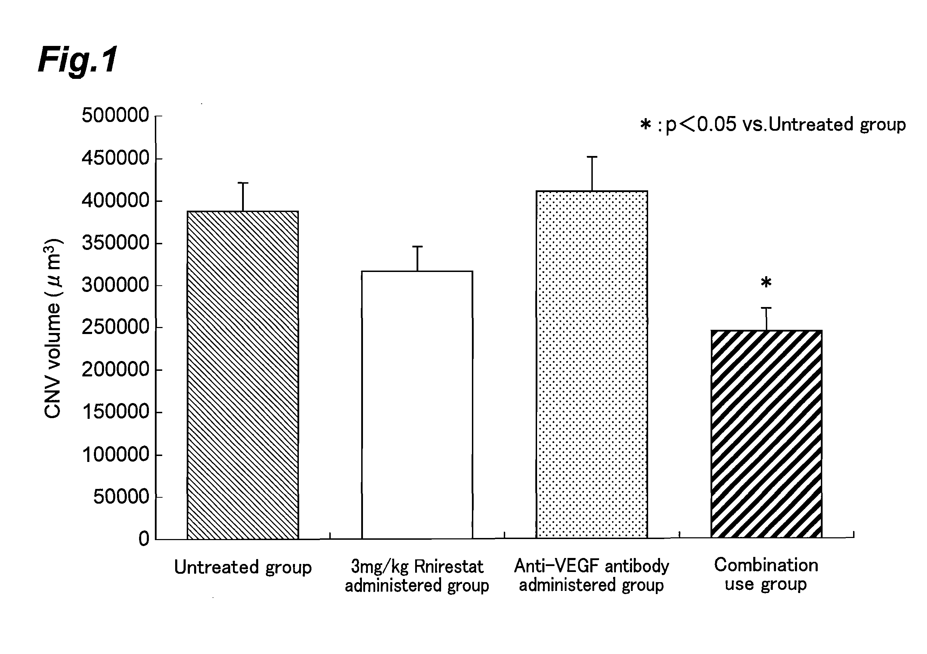

FIG. 1 is a graph showing the choroidal neovascularization (CNV) volume at 7 days after the irradiation of laser. Table 1 shows the CNV suppression rates of the respective groups based on the non-treated group. In the ranirestat-administered group, an effect of suppressing the CNV volume was observed but there was no significant difference as compared to the non-treated group, and the CNV suppression rate was 18.3%. In the anti-VEGF antibody-administered group, no effect of suppressing the CNV volume was observed as compared to the non-treated group. On the other hand, in the combination use group, a significant effect of suppressing the CNV volume was observed as compared to the non-treated group (p<0.05), and a CNV suppression rate twice as high as that of the ranirestat-administered group was shown.

**[Table 1]**

| Test group | CNV suppression rate (%) |
|---|---|
| (b) 3 mg/kg ranirestat-administered group | 18.3 |
| (c) Anti-VEGF antibody-administered group | -6.0 |
| (d) Combination use group | 37.3 |

### Consideration

The present result shows that the effect is enhanced even if ranirestat is used in combination in the state that the clinical effect has attenuated or disappeared due to the decrease of the blood concentration or concentration in tissue of the anti-VEGF antibody. Since the dose of the anti-VEGF antibody at which the maximum medicinal effect is shown is 10 ng, the experiment at this time simulates the state in which the intravitreal concentration of the anti-VEGF agent has decreased to about one-tenth. Accordingly, even in the state in which the effect of the anti-VEGF agent has attenuated or disappeared, the effect of the anti-VEGF agent can be sustained by using ranirestat in combination. Since the enhancement of the effect by combination use extends the sustainability of the effect of the anti-VEGF agent and the interval until readministration due to attenuation of the effect and exacerbation, the enhancement leads to decreasing of the frequency of the administration of the anti-VEGF agent.

The result at this time means that the effect is enhanced by using ranirestat in combination even in the case when the dose of the anti-VEGF agent is decreased. This combination use enables decreasing of the dose of the anti-VEGF agent up to about one-tenth, and thus is expected to lead decreasing of the side effects of the anti-VEGF agent.

Namely, the result in the combination use group at this time further enhances the effectiveness of the anti-VEGF agent whose therapeutic effect has been clinically recognized, and consequently leads to the extension of the time interval until the re-administration of the anti-VEGF agent, i.e., the effect can be sustained. Furthermore, an excellent effect of suppressing angiogenesis can be expected by combination use even in a patient for which the effect of a treatment with an existing anti-VEGF agent is insufficient. Alternatively, it can be expected that a sufficient therapeutic effect is achieved even if the dose of the anti-VEGF agent is decreased.

In addition, since anti-VEGF antibodies that are currently in clinical use are non-selective human-type anti-VEGF agents that inhibit all VEGF isoforms, they are unresponsive to mice. Since the mouse anti-VEGF antibody used in the experiment at this time inhibits all VEGF isoforms in a similar manner to that of an anti-human VEGF antibody, the effect of the mouse anti- VEGF antibody in this test is reflected in the clinical effect of the human anti- VEGF antibody.

### Experiment 2:

### Method

Similarly to Experiment 1, the anti-VEGF antibody and ranirestat were each administered alone, or these were administered in combination, to a mouse that had undergone a treatment to induce CNV. The dose of ranirestat was 10 mg/kg/day, which is the maximum dose used in literature. This dose is considered to be a dose that shows the maximum medicinal effect. The number of the examples was four per one group, and the following four groups were set.
(a) Non-treated group
(b) 10 mg/kg ranirestat-administered group: 10 mg/kg of ranirestat was forcibly and orally administered once a day (10 mg/kg/day)
(c) Anti-VEGF antibody-administered group: 1 ng of an anti-VEGF antibody was administered by intravitreal injection
(d) Combination use group: 1 ng of an anti-VEGF antibody was administered by intravitreal injection + 10 mg/kg of ranirestat was forcibly and orally administered once a day (10 mg/kg/day)

Ranirestat was administered from the day of the laser irradiation (20 to 30 minutes after the irradiation) to seven days after the irradiation. The anti-VEGF antibody was administered by intravitreal injection immediately after the laser irradiation. The CNV volume was measured in a similar manner to Experiment 1.

### Result

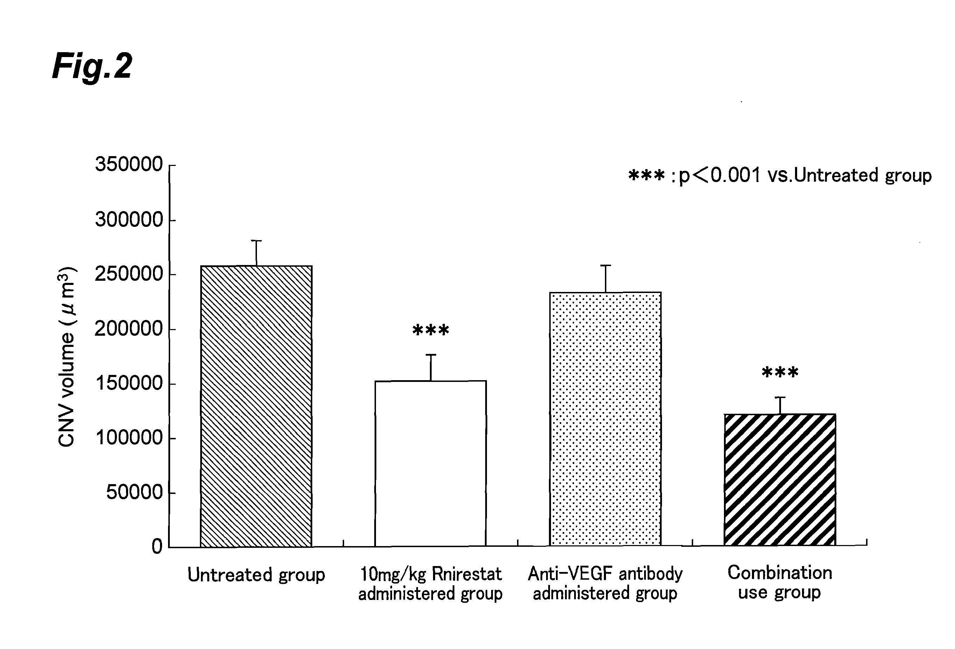

FIG. 2 is a graph showing the choroidal neovascularization (CNV) volume at 7 days after the irradiation of laser. Table 2 shows the CNV suppression rates of the respective groups based on the non-treated group. In the ranirestat-administered group, a significant effect of suppressing the CNV volume was observed (p<0.001) as compared to the non-treated group, and the CNV suppression rate thereof was 39.3%. In the anti-VEGF antibody-administered group, no significant effect of suppressing the CNV volume was observed as compared to the non-treated group. On the other hand, in the combination use group, a significant effect of suppressing the CNV volume was observed as compared to the non-treated group (p<0.001), and the CNV suppression rate thereof was 53.5%.

**[Table 2]**

| Test group | CNV suppression rate (%) |
|---|---|
| (b) 10 mg/kg ranirestat-administered group | 39.3 |
| (c) Anti-VEGF antibody-administered group | 10.0 |
| (d) Combination use group | 53.5 |

### Consideration

In the combination use group of the anti-VEGF antibody and ranirestat, a higher effect of suppressing the CNV volume than that of the ranirestat-administered group was observed. Since it is considered that the ranirestat-administered group showed the maximum effect against suppression of angiogenesis, it can be said that the combination use group showed an effect of suppressing angiogenesis that goes beyond the maximum effect of ranirestat. Furthermore, in the combination use group, an effect of suppressing angiogenesis that goes beyond the maximum effect of ranirestat was shown by the combination use of the anti-VEGF antibody whose effect had attenuated with ranirestat. Since an effect of suppressing angiogenesis was able to be obtained, it is strongly suggested that an effect of suppressing increased vascular permeability can also be obtained. These results show that the effect that was not able to be achieved by ranirestat alone even if the dose of ranirestat was increased was able to be achieved by the combination use. Such effect that is equal to or more than the maximum medicinal effect goes beyond the expectation of persons skilled in the art.

## Claims

1. A pharmaceutical for preventing or treating a disorder accompanied by ocular angiogenesis and/or increased ocular vascular permeability composed of a combination of:
an anti-VEGF agent; and
a pyrrolo[1,2-a]pyrazine derivative represented by a general formula (I): 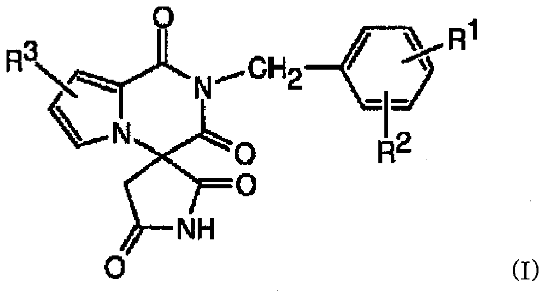 in the formula, R¹ and R² are simultaneously or individually each a hydrogen atom, a halogen atom, a trifluoromethyl group, a lower alkyl group, a lower alkoxy group or a nitro group, and R³ is a hydrogen atom, a halogen atom or a lower alkyl group, or a pharmacologically acceptable salt thereof.

2. A pharmaceutical for preventing or treating a disorder accompanied by ocular angiogenesis and/or increased ocular vascular permeability, comprising a pyrrolo[1,2-a]pyrazine derivative represented by a general formula (I): 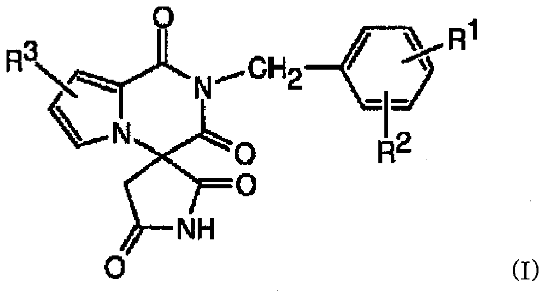 in the formula, R¹ and R² are simultaneously or individually each a hydrogen atom, a halogen atom, a trifluoromethyl group, a lower alkyl group, a lower alkoxy group or a nitro group, and R³ is a hydrogen atom, a halogen atom or a lower alkyl group, or a pharmacologically acceptable salt thereof as an active ingredient, and
the pharmaceutical being administered in combination with an anti-VEGF agent.

3. The pharmaceutical according to claim 1 or 2, wherein the pyrrolo[1,2-a]pyrazine derivative represented by the general formula (I) is
(3R)-2'-(4-bromo-2-fluorobenzyl)spiro[pyrrolidine-3,4'(1'H)pyrrolo[1, 2-a]pyrazine]-1',2,3',5(2'H)-tetrone.

4. The pharmaceutical according to any one of claims 1 to 3,
wherein the anti-VEGF agent is an intravitreal injection drug.

5. The pharmaceutical according to claim 4, wherein the intravitreal injection drug is selected from the group consisting of an anti-VEGF antibody, a VEGF Ligand inhibitor, a VEGF receptor antagonist and a nucleic acid drug related to VEGF.

6. The pharmaceutical according to any one of claims 1 to 5,
wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy and neovascular glaucoma.

7. A kit comprising:
the pharmaceutical according to claim 2; and
a written instruction describing a method for administering the pharmaceutical in combination with an anti-VEGF agent for preventing or treating a disorder accompanied by ocular angiogenesis and/or increased ocular vascular permeability.
